# EUROPEAN PATENT SPECIFICATION

(11) **EP 1 307 747 B1**
(45) Date of publication and mention of the grant of the patent: **11.11.2009**
(21) Application number: 01954334.7
(22) Date of filing: 23.07.2001
(51) Int. Cl.: G01N 33/574

(54) **Diagnostic method for liver cancer based on SCCA1 proteins**
DIAGNOSTISCHES VERFAHREN FÜR LEBERKREBS AUF BASIS DES SCCA1-PROTEINS
DIAGNOSTIQUE DU CANCER DU FOIE BASE SUR LA DETECTION DE PROTEINES SCCA1

(30) Priority: 28.07.2000 IT PD000192
(43) Date of publication of application: 07.05.2003
(73) Proprietor: Xeptagen S.p.A., 80100 Napoli (IT)
(72) Inventor: PONTISSO, Patrizia, I-35100 Padova (IT); SITIA, Felice, I-20121 Milano (IT)
(74) Representative: Gustorf, Gerhard
(86) International application number: PCT/IT2001/000393
(87) International publication number: WO 2002/010757

(56) References cited:
- CATALTEPE SULE ET AL: "Development of specific monoclonal antibodies and a sensitive discriminatory immunoassay for the circulating tumor markers SCCA1 and SCCA2." CLINICA CHIMICA ACTA, vol. 295, no. 1-2, May 2000 (2000-05), pages 107-127, XP002198888 ISSN: 0009-8981
- MITSUMORI M ET AL: "SQUAMOUS CELL CARCINOMA ANTIGEN AS TUMOR MARKER FOR HEAD AND NECK MALIGNANCY" KAWASAKI MEDICAL JOURNAL, vol. 13, no. 1, 1987, pages 19-24, XP001073687 ISSN: 0385-0234

## Description

The need for new and accurate diagnostic methods in the oncology field is increasing, especially for tumor diseases with high incidence and where the rapidity of diagnosis is essential for the patient life.

Hepatocarcinoma (HCC) represents one of the most widespread diseases in the world and its prognosis is particularly severe since only about 3% of patients survive after 5 years. A problem related to this disease is the difficulty to have an early diagnosis in order to carry out a timely intervention.

Currently, alpha-Fetoprotein is the only serologic parameter used in diagnostic screening of hepatocarcinoma, even if its specificity and sensitivity are not satisfactory. In fact, the values of alpha-Fetoprotein can be slightly elevated for patients with hepatic cirrhosis without tumor, or can be normal also in the presence of isolated tumoral nodules that can be evidenced by ultrasound examinations. For this reason it is recommended to screen patients with cirrhosis by liver ultrasonography every 6 months. Major limitation of this method are related to the difficult technical detection of tumors with 1 cm of diameter and tumors with the same density as normal liver tissue which are visible only with additional cunning devices not routinely employed.

The accurate diagnosis of chronic hepatitis is obtained by liver biopsy, since other clinical and serological tests do not assure definitive results. However also this procedure has some limits, since experienced personnel is required and the results interpretation as well as the etiology distinction (HBV/drugs) are not easy to perform. The availability of reagents, able to detect typical markers of the liver malignant evolution, might allow an easier diagnosis. Antibodies specific for immunohistochemistry to diagnose hepatocarcinoma should be warmly accepted by clinicians in order to get accurate and definitive diagnosis even in non specialized centers.

SCCA has been described previously as tumor marker for head and neck carcinoma (Mitsumori et. A. Kawasaki Medical J. 13: 19-24, 1987). SCCA is physiologically found in the spinous and granular layers of normal squamous epithelium and is typically over expressed in neoplastic cells of epithelial origin. The expression of SCCA1 in a non-squamous tissue such as liver tissue has not been reported before in the literature.

Surprisingly, it has been found that in hepatic tissues characterized by malignant transformations, an over-production of a protein, the squamous cell carcinoma antigen 1 (SCCA1), takes place.

Also other closely related proteins, such as SCCA2, may be overproduced.

Therefore, a first aspect of this invention relates to a method as defined in claim 1.

A second aspect of this invention relates to a method of defined in claim 2.

A typical method to prepare polyclonal antibodies specific for the SCCA1 protein includes:
1] Immunizing an appropriate animal (rabbit, sheep, goat, rat, mouse) with SCCA1 protein mixed with an appropriate adjuvant, and antigen injection; it may be useful to conjugate the SCCA1 protein to a carrier-like albumin or keyhole limpet hemocyanin.
2] Taking serum samples after an appropriate time to check the production of specific antibodies in the serum fraction.
3] Antibodies purification by column chromatography or precipitation techniques.

All of these techniques mentioned above are described in the literature and are known by experts in this field, and can be used also for the production of polyclonal antibodies against SCCA1 related proteins.

A typical example of a method for anti-SCCA1 monoclonal antibody preparation includes:
1. Immunization of an appropriate animal like mouse or rat with the SCCA1 protein as antigen and collection of splenocytes
2. Generation of hybridomas by fusion between non-secreting myeloma cells and mouse splenocytes
3. Screening hybridomas secreting antigen-specific antibodies.
4. Growing hybridomas selected for antibody production.
5. Antibody purification by conventional methods.

The screening procedure referred to point 3 is carried out by:
1] Immobilization of SCCA1 protein, either alone or conjugated with other molecules, on plastic microtiter plates compatible for ELISA assays, or beads usable for diagnostic purposes
2] Incubation of immobilized SCCA1 with the antibody sample
3] Washing of the immobilized protein/antibody complex
4] Determination of adsorbed antibody by appropriate reagents.

These methods are reported in the literature ("Immunochemistry in Practice", Johnstone & Thorpe, (1987) Blackwell, Oxford, UK, for example) and are well known by experts in this field, and may be applied to the generation of monoclonal antibodies against SCCA1 related proteins.

Preferably, phase 1 is carried out using 96 well microtiter plates made of PVC, where each well is filled with a solution of 0.1 M bicarbonate buffer, pH 9, containing different amounts (0 - 50 micrograms) of SCCA1 protein. After 24 hours incubation, the solution is removed, microtiter plates are washed with phosphate buffer and wells filled with a solution containing 3% bovine albumin to block the uncoated plastic surface. In the phase 2, microtiter plates are washed with phosphate buffer and wells filled with samples containing the antibodies to be determined. Microtiter plates are then incubated at 37°C for 4 hours. Washing in the phase 3 is preferably performed with phosphate buffer. Finally, phase 4 is carried out by adding an anti-immunoglobulin antibody (for example a rabbit anti-human immunoglobulin antibody solution) which is conjugated to horseradish peroxidase. After 2 hours of incubation plates are washed again with phosphate buffer. A solution of o-phenylenediamine dihydrochloride is added and the color developed is determined with an appropriate ELISA plate reader. All these procedures referred above are widely described in the literature and are known by experts in this field.

A typical example for a method to detect SCCA1 protein on liver tissues includes:
1] Obtaining liver tissue by hepatic biopsy
2] Tissue incubation with a solution of poly- or monoclonal antibody specific for SCCA1 protein, or closely related proteins.
3] Washing out exceeding antibody
4] Adsorbed antibody detection by other specific antibodies; such specific antibodies can be conjugated with enzymes or chromogenic or fluorescent or radioactive or chemioluminescent compounds or other systems for detection known from literature.

All the procedures referred above are well described in the literature and are known by experts in this field.

The invention will be described by the following examples.

### EXAMPLE 1. Production of SCCA1-specific polyclonal antibodies developed in rabbits.

### Recombinant or extractive SCCA1 protein was used to immunize New Zealand White rabbits by intradermal injections. A boost with the same antigen was carried out after four weeks and after about 2 months serum samples were taken in order to obtain the serum fraction containing specific antibodies. Such fraction was assayed for the presence of specific anti-SCCA1 antibodies by ELISA technique.

Microtiter plates for ELISA assay (Falcon Cat No. 3912) were coated with a solution of SCAA1 at a concentration between 0 and 50 µg/ml in bicarbonate buffer pH 9.0 for 12 hours at 4°C (100 µl). Plates were washed with 150 mM sodium phosphate buffer, pH 7.2 (PBS) (200 µl), and then each well was filled with 200 µl of PBS solution containing 3 % of bovine serum albumin (BSA, Sigma Cat. No. A-4503).

After 1 hour incubation, plates were washed four times with PBS and each well filled with anti-SCCA1 rabbit antibody solution (100 µl) at concentrations between 0 and 20 µg/ml. After incubation for 2 hours at 37°C, plates were washed again with PBS for eight times and then 100 µl of horseradish peroxidase-labeled goat anti-rabbit immunoglobulins (Sigma Cat. No. A-6154) solution, diluted 1000-fold with PBS-3%BSA, was added to each well. After 1 hour of incubation plates were washed again with PBS. Then wells were filled with 100 µl of 1 mg/ml o-phenylendiamine dihydrochloride (Sigma P 6912) dissolved in 0.1 M sodium citrate buffer containing 5 mM hydrogen peroxide. The color was allowed to develop for 30 min and then the reaction was stopped by adding 25 µl of 4.5 M sulfuric acid solution. Absorbance at 492 nm was determined by an ELISA plate reader (Merck, Mod. MIOS).

### EXAMPLE 2. Purification of polyclonal and monoclonal antibodies by affinity chromatography on SCCA1-immobilized columns.

SCCA1 protein of recombinant or extractive origin (5 mg), was dissolved in 5 ml of 0.1 M sodium bicarbonate buffer, pH 9.0, and then added to 1.2 g of NHS-Activated Sepharose 4 Fast Flow (Amersham Pharmacia Biotech, Code No. 17-0906-01) matrix designed for affinity chromatography suitable for peptide and protein immobilization. The suspension was mixed for 4 hours and coupling extent was onitored taking samples at different time and analyzing them by RP-HPLC. About 85 % of the initial protein was attached to the matrix by formation of stable bonds after 4 hours. Derivatized resin was washed with 50 ml of a solution of 1 M TRIS pH 9.0, and then packed in a 100 x 6.6 mm I.D column. For purification procedures, columns were equilibrated with 50 mM TRIS pH 7.0, at a flow rate of 1.0 ml/min monitoring the effluent by UV at 280 nm. One milliliter of crude rabbit serum containing anti-SCCA1 antibodies was applied to the column and after washing out of unbound proteins, buffer applied was changed to 0.1 M acetic acid. The adsorbed proteins were collected and analyzed by SDS-polyacrylamide gel electrophoresis. Monoclonal antibodies against SCCA1 protein were purified in the same manner loading the column with samples from cell culture supernatants obtained from hybridomas, using the same elution procedures described above.

### EXAMPLE 3. Monoclonal antibody production

Four weeks old Balb/c mice (Harlan Nossan, Milano, Italia) were immunized with 50 µg of SCCA1 protein of recombinant or extractive origin. In the following two months, animals were boosted every two weeks with the same amount of protein. After each boost anti-SCCA1 antibody titer was monitored by ELISA assay using microtiter plates coated with SCCA1, and on the basis of immunoreactivity one mouse was chosen for hybridomas preparation at the end of the immunization procedure.

Murine myeloma cells used for hybridomas preparation were NS0 cultured in the following conditions [Reagents were purchased from Sigma-Aldrich, Milano, Italia: DMEM (cat. n. D5671) + 10% FBS (cat. n. F7524) + 1% NEAA (cat. n. M7145) + 2mM Glutamine (cat. n. G7513) + Penicillin/Streptomycin (cat. n. P0871) 100U/ml].

NS0 cells do not secrete any antibody and are negatively selected with HAT medium.

The mouse with the highest titer was selected and sacrificed in a CO₂ chamber. Spleen was aseptically removed, placed in a tissue culture dish and washed with 10 ml of DMEM-GM. Thus, the spleen was mechanically broken up and the obtained suspension was placed in a tissue culture dish containing 5 ml of DMEM-GM pre-warmed at 37°C. The remaining clumps were broken up with a 5 ml pipette, and cells were recovered by centrifugation, washed with the same medium and counted by a Burker chamber.

NS0 cells were counted and resuspended in DMEM-GM medium, and added to splenocytes in a 1:5 ratio. The cell mixture was recovered by centrifugation, washed again with DMEM-GM medium, and in order to enhance fusion, 1 ml of DMEM-GM medium containing 44% PEG (Sigma-Aldrich, Milano, Italia, cat. n. P7777) was added to the pellet in 1 min. Two milliliters of the same medium were added in the next 3 min, and after pellet resuspension, 7 ml of DMEM-GM + 10% were added under stirring. Cells were recovered by centrifugation and gently resuspended with 10 ml of DMEM-HAT [DMEM-GM + 15% FCS + 2% HAT 50x (Sigma-Aldrich, Milano, Italia, cat. n. H0262) + 10% ECGS (Sigma-Aldrich, Milano, Italia, cat. n. E-0760) + Penicillin/Streptomycin 100U/ml]; the cell mixture was diluted and dispensed in 10 microtiter plates.

After about 15 days clone supernatants were analyzed by ELISA assay in order to evaluate SCCA1-specific antibody production by the method described in example 1. Positive clones were amplified, and secreted monoclonal antibodies were characterized and the isotype determined. Supernatants were purified by the method described in example 2.

### EXAMPLE 4.

Patients with primary liver cancer deriving from various etiology (HBV, HCV, alcohol, hemochromatosis ...) as diagnosed by histological examination were selected, including asymptomatic patients positive to occasional ultrasonography and patients in a condition of advanced disease and intrahepatic massive cancer diffusion or extrahepatic metastases. Since liver cancer is associated with several risk factors, patients with active or latent HBV infection, with HCV infection, or with hemochromatosis diagnosed at molecular level were also included.

Detection of SCCA 1 protein on liver tissues. SCCA 1 protein was determined on hepatic formalin fixed tissues from hepatic biopsies and from US-guided needle aspiration of nodular lesions evidenced in the liver of patients. In order to detect SCCA 1 protein, the monospecific rabbit polyclonal antibody directed against SCCA protein, produced as described in example 1, was used. The antigen/antibody interaction was detected by the avidin-biotin complex (ABC) method, after endogenous biotin activity blocking. Biopsies from normal liver tissues and from mouse liver were used as negative controls. As positive controls, primary liver cancer samples obtained from surgical operation were used, a portion of which was embedded in paraffin blocks for histological and immunohistochemical analysis, whereas the remaining were stored in liquid nitrogen. This frozen material was used to control the mRNA expression of SCCA protein by PCR analysis and detection by gel electrophoresis and ethidium bromide staining. In order to set up the immunohistochemical method for SCCA detection on embedded tissue, tissues resulting positive to the presence of SCCA mRNA by PCR technique were chosen. The method was based on incubation of embedded slides with specific rabbit polyclonal antibody, followed by a second incubation with a specific anti rabbit alkaline phosphatase - labelled polyclonal antibody and a final detection with fuchsin. Experiments were positive when red granuli were formed in the cellular cytoplasm (Fig. 1). All samples (100%) positive by PCR determination resulted positive to the presence of SCCA by the immunohistochemical method as described above. Likewise, all samples negative by SCCA mRNA detection resulted negative also by immunohistochemical analysis, similarly to normal liver tissues used as negative control.

Similar results were obtained employing monoclonal antibodies, and substituting the secondary antibody with antibodies against murine immunoglobulins in the staining procedure.

## Claims

1. Method for diagnosing hepatocellular carcinoma, **characterized in that** it comprises the measurement of SCCA1 protein with antibodies against SCCA1, in hepatic biopsy.

2. Method according to claim 1, **characterized in that** it comprises the use of polyclonal antibodies.

3. Method according to claim 2, **characterized in that** it comprises the use of antibodies generated in rabbits.

4. Method according to claim 1, **characterized in that** it comprises the use monoclonal antibodies.

5. Method according to claim 4, **characterized in that** said antibodies are generated in mice.

6. Method according to claim 1, **characterized in that** it comprises:
- treatment of hepatic tissue with antibodies according to claim 1,
- removal of exceeding antibody by washing,
- detection of adsorbed antibody by antibodies specific for immunoglobulins according to claim 1, wherein said antibodies are conjugated with chromogenic reagents, or fluorescent labeling reagents, and by analysis at the optical microscope.

## Patentansprüche

1. Verfahren zur Diagnose von hepatozellulären Karzinomen, **gekennzeichnet durch** die Messung von SCCA1-Protein mit Antikörpern gegen SCCA1 in einer Hepatobiopsie.

2. Verfahren nach Anspruch 1, **gekennzeichnet durch** die Verwendung von polyklonalen Antikörpern.

3. Verfahren nach Anspruch 2, **gekennzeichnet durch** die Verwendung von in Kaninchen erzeugten Antikörpern.

4. Verfahren nach Anspruch 1, **gekennzeichnet durch** die Verwendung von monoklonalen Antikörpern.

5. Verfahren nach Anspruch 4, **gekennzeichnet durch** die Verwendung von in Mäusen erzeugten Antikörpern.

6. Verfahren nach Anspruch 1, **dadurch gekennzeichnet, dass** dieses umfasst:
- Behandeln des hepatitschen Gewebes mit Antikörpern gemäß Anspruch 1,
- Entfernen der überschüssigen Antikörper durch Waschen,
- Ermitteln der adsorbierten Antikörper durch für Immunglobuline spezifische Antikörper nach Anspruch 1, wobei diese Antikörper konjugiert werden mit chromogenen Reagenzien oder Fluoreszenz markierenden Reagenzien, und durch Analyse am optischen Mikroskop.

## Revendications

1. Procédé pour le diagnostic du cancer hépatocellulaire, **caractérisé par le fait qu'**il comprend le mesurage des protéines SCCA1 par les anticorps des SCCA1 dans une biopsie du foie.

2. Procédé selon la revendication 1, **caractérisé par le fait qu'**il comprend l'utilisation d'anticorps polyclonaux.

3. Procédé selon la revendication 2, **caractérisé par le fait qu'**il comprend l'utilisation d'anticorps générés dans des lapins.

4. Procédé selon la revendication 1, **caractérisé par le fait qu'**il comprend l'utilisation d'anticorps monoclonaux.

5. Procédé selon la revendication 4, **caractérisé par le fait qu'**il comprend l'utilisation des anticorps monoclonaux générés dans des souris.

6. Procédé selon la revendication 1, **caractérisé par le fait qu'**il comprend:
- le traitement du tissu hépatique avec des anticorps selon la revendication 1,
- l'élimination des anticorps excédentaires par lavage,
- la détection des anticorps adsorbés par des anticorps spécifiques pour des immunoglobulines selon la revendication 1, lesdits anticorps étant conjugués avec des réactifs chromogènes ou réactifs marqueurs fluorescents, et par l'analyse au microscope optique.
